# EUROPEAN PATENT APPLICATION

(11) **EP 4 198 021 A1**
(43) Date of publication of application: **21.06.2023**
(21) Application number: 21856055.5
(22) Date of filing: 24.06.2021
(51) Int. Cl.: C07D 211/14, A61K 31/451, A61K 9/00, A61P 25/28

(54) **DONEPEZIL ETHER PALMITATE OR PHARMACEUTICALLY ACCEPTABLE SALT THEREOF**

(30) Priority: 14.08.2020 KR 20200102535
(71) Applicant: Chong Kun Dang Pharmaceutical Corp., Seoul 03742 (KR)
(72) Inventor: NAM, Dong Hyuk, Yongin-si Gyeonggi-do 16995 (KR); LEE, Jaemin, Yongin-si Gyeonggi-do 16995 (KR); PARK, So Hyun, Yongin-si Gyeonggi-do 16995 (KR); KANG, Sung Kwon, Yongin-si Gyeonggi-do 16995 (KR); PARK, Shin Jung, Yongin-si Gyeonggi-do 16995 (KR)
(74) Representative: D Young & Co LLP
(86) International application number: PCT/KR2021/007936
(87) International publication number: WO 2022/035048

(57) **Abstract**

The present invention relates to novel donepezil ether palmitate or a pharmaceutically acceptable salt thereof; and a sustained-release pharmaceutical composition containing same as a main ingredient. Since an ether palmitate group is introduced to donepezil, the donepezil ether palmitate of the present invention reduces the initial release of donepezil, which is the active ingredient, when administered into the body, so as to reduce the risk of side effects such as drug toxicity, and allows donepezil to be uniformly released in the body over a long time to increase drug treatment effects for dementia patients.

## Description

### [Technical Field]

The present disclosure relates to a novel donepezil ether palmitate or a pharmaceutically acceptable salt thereof, and a sustained-release pharmaceutical composition comprising the same as a main ingredient. Specifically, the present disclosure relates to a donepezil ether palmitate or a pharmaceutically acceptable salt, and an injectable composition comprising the same, capable of maintaining a constant blood concentration of the active ingredient, donepezil, and supplying the active ingredient over a long time without risk of side effects when administered in the body.

### [Background Art]

Dementia refers to a disease associated with complex cognitive impairment showing memory loss, deterioration of intelligence, personality change, behavioral abnormalities, and the like. In other words, dementia is a degenerative cranial nerve disease that is caused by irreversible dysfunction in neural networks due to the death of slow nerve cells causing central nervous system degenerative diseases, and eventually causes permanent loss of human body functions.

The mechanism of dementia has not been fully elucidated, but it is known that choline acetyltransferase (hereinafter referred to as ChAT), which synthesizes acetylcholine (hereinafter referred to as ACh), is reduced by about 20 to 30%, and that the concentration of acetylcholine, a neurotransmitter, is reduced by about 16 to 30% in the brains of dementia patients compared to normal people. As a result of the above study, studies using an inhibitor that inhibits acetylcholinesterase (hereinafter referred to as AChE), which is an enzyme that hydrolyzes acetylcholine, a neurotransmitter, as an indirect treatment method, have been conducted.

Acetylcholinesterase is an enzyme that hydrolyzes acetylcholine, one of the neurotransmitters that mediate the activity of parasympathetic nerves in the body, into choline and acetate, and is formed in the endoplasmic reticulum membrane and moves to the cell membrane to perform function thereof. The enzyme is an important enzyme that is most distributed in the cholinergic nerve and surroundings thereof, especially in the neuromuscular junction, and found in plasma, liver and also other tissues.

Most of therapeutic agents for dementia that are currently used are acetylcholinesterase inhibitors, which include Donepezil (trade name: Aricept), Tacrine (trade name: Cognex), Rivastigmine (trade name: Exelon), Galantamine (trade name: Reminyl), and the like.

A first-generation acetylcholinesterase inhibitor includes tacrine, which is a drug first approved as an anti-dementia therapeutic agent. However, tacrine is required to be administered 4 times a day due to a short duration of action, and has a problem of causing liver toxicity.

A second-generation acetylcholinesterase inhibitor includes Donepezil, which is a compound represented by the following Chemical Formula. Donepezil was approved in the United States for a treatment of dementia in 1996, and is known as a therapeutic agent for mild and moderate or more severe Alzheimer's disease, most of which is orally administered in the form of tablets.

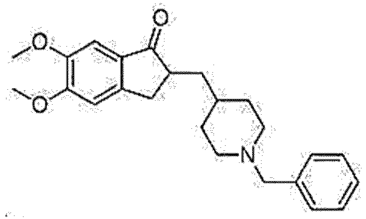

However, it is known that oral administration of donepezil tablets causes gastrointestinal side effects such as diarrhea, nausea, loss of appetite, muscle convulsions, and the like, in some patients. In addition, the oral preparations of donepezil hydrochloride that are currently commercially available are generally administered at a starting dose of 5 mg once a day at bedtime and used for 4 to 6 weeks, and then the dose is increased to 10 mg once a day. However, the treatment method in such a manner has a disadvantage of low medication compliance because the preparations have to be orally administered daily, particularly in patients with dementia.

In recent years, oral disintegrating tablets are commercially available for patients who have difficulty in swallowing. Further, when it is difficult to perform oral administration, transdermal administration of an ointment preparation has been proposed (Japanese Laid-open Publication (Hei) No. 11-315016). In addition, ointments, suppositories, and the like, have been suggested to solve cases where it is difficult to take medicine orally in a state in which dementia symptoms are significantly advanced. However, these formulations also have a problem in that active ingredients must be continuously administered over a long period of time.

In order to maintain a stable drug concentration for a long period of time while improving patient convenience and compliance by reducing the frequency of drug administration, there is a method of formulating a sustained-release injection. However, when administering a sustained-release injection, it is very difficult to ensure that the drug is continuously and uniformly released over a long period of time while maintaining a biological activity thereof in the body.

In particular, since the sustained-release injections have a high initial burst release, which may cause side effects including toxic reactions, it is necessary to eliminate or at least minimize the high initial burst release.

Therefore, there is a need for development of a preparation capable of inhibiting the high initial burst release of donepezil and continuously releasing donepezil for a long period of time.

Accordingly, the present inventors developed a donepezil ether palmitate in which an ether palmitate group is introduced into donepezil to be capable of continuously and uniformly releasing donepezil in the body while reducing the high initial burst release of donepezil, and completed the present disclosure.

### [Disclosure]

### [Technical Problem]

An object of the present disclosure is to provide donepezil ether palmitate or a pharmaceutically acceptable salt thereof, and a pharmaceutical composition containing the same, capable of improving drug treatment compliance of dementia patients with low risk of side effects when administered into the body.

Specifically, the present disclosure provides donepezil ether palmitate or a sustained-release pharmaceutical composition containing the same capable of reducing the initial burst release of donepezil to reduce the risk of side effects such as drug toxicity, and allowing donepezil to be uniformly released in the body over a long time to increase drug treatment effects for dementia patients.

### [Technical Solution]

The present disclosure provides a compound represented by the following Chemical Formula 1 or a pharmaceutically acceptable salt thereof:

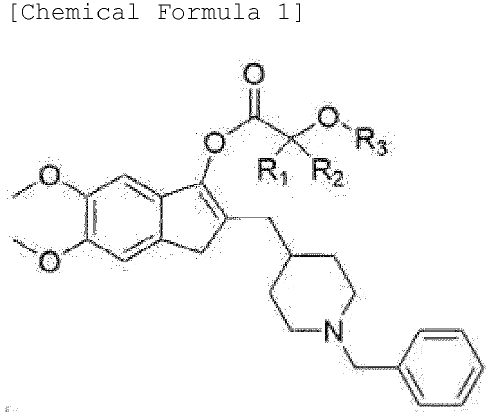

in the Formula above,
R₁ and R₂ are each independently hydrogen, halogen, hydroxy, C₁-C₆ alkyl, C₁-C₆ haloalkyl, C₁-C₆ alkoxy, or aryl; and
R₃ is C₁₂-C₁₆ alkyl.

Further, the present disclosure provides a sustained-release pharmaceutical composition for preventing or treating dementia, comprising the compound represented by Chemical Formula 1 or a pharmaceutically acceptable salt thereof.

Further, the present disclosure provides 2-((1-benzylpiperidin-4-yl)methyl)-5,6-dimethoxy-1H-inden-3-yl 2-(tetradecyloxy)acetate represented by the following Chemical Formula 2 or a pharmaceutically acceptable salt thereof:

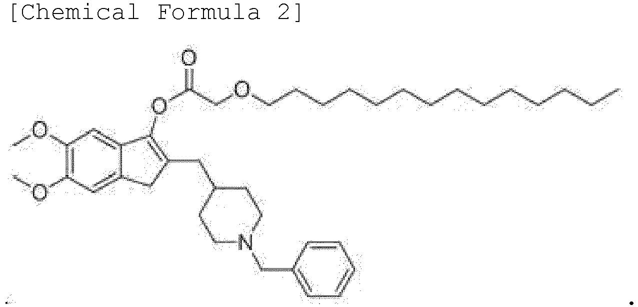

Further, the present disclosure relates to a sustained-release pharmaceutical composition for preventing or treating dementia, comprising the compound represented by Chemical Formula 2 or a pharmaceutically acceptable salt thereof.

The chemical name of the compound represented by Chemical Formula 2 is "2-((1-benzylpiperidin-4-yl)methyl)-5,6-dimethoxy-1*H*-inden-3-yl 2-(tetradecyloxy)acetate" and in the present disclosure, the compound in which an ether palmitate group is introduced into donepezil according to the present disclosure is referred to as donepezil ether palmitate (DEP).

The donepezil ether palmitate of the present disclosure may be prepared by reacting donepezil free base with 2-(tetradecyloxy)acetyl chloride.

The 2-(tetradecyloxy)acetyl chloride may be prepared by comprising: (i) mixing 1-tetradecanol, sodium chloroacetate, and potassium hydroxide to obtain sodium 2-(tetradecyloxy)acetate; (ii) reacting the sodium 2-(tetradecyloxy)acetate with an aqueous HCl solution to obtain 2-(tetradecyloxy)acetic acid; and (iii) reacting the 2-(tetradecyloxy)acetic acid with oxalyl chloride.

The pharmaceutical composition of the present disclosure may be formulated as a preparation for parenteral administration, for example, intramuscular injection, intravenous injection, subcutaneous injection, intradermal injection, or intravenous drip infusion, preferably as a preparation for intramuscular injection.

The pharmaceutical composition of the present disclosure is preferably administered every 2 to 20 weeks, more preferably every 4 to 16 weeks.

### [Advantageous Effects]

The donepezil ether palmitate or a pharmaceutically acceptable salt thereof, and the sustained-release pharmaceutical composition containing the same as a main ingredient of the present disclosure may have a low initial burst release of donepezil after administered into the body, thereby minimizing the risk of side effects including toxic reactions, and may maintain an effective concentration of donepezil in blood over a long time to exhibit a therapeutic effect even with a single administration, thereby improving the drug treatment compliance of dementia patients.

### [Description of Drawings]

FIG. 1 shows concentration of donepezil in blood measured after administration of donepezil (D) to rats.
FIG. 2 shows concentration of donepezil in blood measured after administration of donepezil ether palmitate (DEP) to rats.

### [Best Mode]

### Definition

As used herein, "alkyl" is a hydrocarbon having primary, secondary, tertiary, or quaternary carbon atoms, and includes saturated aliphatic groups that may be straight-chain, branched, or cyclic, or combinations thereof. For example, an alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Examples of suitable alkyl groups may include, but are not limited to, methyl (Me, -CH₃) , ethyl (Et, -CH₂CH₃), 1-propyl (n-Pr, n-propyl, - CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH (CH₃)CH₂CH₂CH₃), 3-pentyl (-CH (CH₂CH₃)₂), 2-methyl-2-butyl (-C(CH₃)₂CH₂CH₃), 3-methyl-2-butyl (-CH (CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH (CH₃)CH₂CH₂CH₂CH₃), 3-hexyl (-CH (CH₂CH₃)(CH₂CH₂CH₃)), 2-methyl-2-pentyl (-C (CH₃)₂CH₂CH₂CH₃), 3-methyl-2-pentyl (-CH (CH₃)CH(CH₃)CH₂CH₃), 4-methyl-2-pentyl (-CH (CH₃)CH₂CH(CH₃)₂), 3-methyl-3-pentyl (-C(CH₃)(CH₂CH₃)₂), 2-methyl-3-pentyl (-CH (CH₂CH₃)CH (CH₃)₂), 2,3-dimethyl-2-butyl (-C(CH₃)₂CH(CH₃)₂), 3,3-dimethyl-2-butyl (-CH (CH₃)C (CH₃)₃), and octyl (-(CH₂)₇CH₃).

"Alkoxy" refers to a group having the moiety -O-alkyl in which the alkyl group as defined above is attached to the parent compound through an oxygen atom. The alkyl moiety of the alkoxy group may have, for example, 1 to 20 carbon atoms (i.e. C₁-C₂₀ alkoxy), 1 to 12 carbon atoms (i.e. C₁-C₁₂ alkoxy), 1 to 10 carbon atoms (i.e. C₁-C₁₀ alkoxy), or 1 to 6 carbon atoms (i.e. C₁-C₆ alkoxy). Examples of suitable alkoxy groups may include, but are not limited to, methoxy (-O-CH₃ or -OMe), ethoxy (-OCH₂CH₃ or -OEt), and t-butoxy (-OC(CH₃)₃ or -O-tBu) .

As used herein, the terms "halo" and "halogen" mean halogen and include chloro, fluoro, bromo, and iodo.

A "haloalkyl" is an alkyl group in which at least one of hydrogen atoms of the alkyl group as defined above is replaced by a halogen atom. The alkyl moiety of the haloalkyl group may have, for example, 1 to 20 carbon atoms (i.e. C₁-C₂₀ haloalkyl), 1 to 12 carbon atoms (i.e. C₁-C₁₂ haloalkyl), 1 to 10 carbon atoms (i.e. C₁-C₁₀ haloalkyl), or 1 to 6 carbon atoms (i.e. C₁-C₆ haloalkyl). Examples of the haloalkyl groups may include, but are not limited to, -CF₃, -CHF₂, -CFH₂, and -CH₂CF₃.

"Aryl" includes monocyclic, bicyclic or polycyclic, substituted or unsubstituted, monovalent or divalent aromatic hydrocarbon groups in which each atom of the ring is carbon. Preferably, the aryl ring is a 6- to 20-membered ring, a 6- to 14-membered ring, a 6- to 10-membered ring, or more preferably a 6-membered ring. The aryl group may be a polycyclic ring system having two or more cyclic rings in which two or more carbons are common to two adjacent rings, wherein at least one of the rings is aromatic and the other cyclic rings may be, for example, cycloalkyl, cycloalkenyl, cycloalkynyl, aryl, heteroaryl, and/or heterocycloalkyl. The aryl group may include benzene, naphthalene, phenanthrene, anthracene, indene, indane, phenol, aniline, and the like.

The term "Cx-y" or "Cx-Cy" when used with a chemical moiety such as alkyl, haloalkyl, or alkoxy, is meant to include groups containing from x to y carbons in the chain. C₀alkyl represents hydrogen if the group is at the end of the carbon chain or represents a bond if it is inside the chain. For example, a C₁-C₂₀ alkyl group contains 1 to 20 carbon atoms in the chain.

Hereinafter, the present disclosure will be described in more detail through the following Examples. However, these Examples are only provided for illustrative purposes to facilitate understanding of the present disclosure, and the scope of the present disclosure is not limited by the following examples.

### [Example 1]

### Synthesis of 2-(tetradecyloxy)acetyl chloride (EPS-Cl)

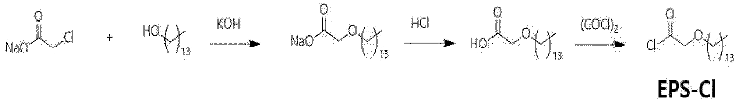

### 1) Preparation of sodium 2-(tetradecyloxy)acetate (EPS-Na)

To the reaction vessel, 400 g of 1-tetradecanol was added and dissolved at 60°C, and then 108.7 g of sodium chloroacetate and 108 mL of n-heptane were added. To the reaction solution, 78.5 g of potassium hydroxide was added, and the mixture was heated to 85°C and stirred for 3 hours. Into the reaction solution, 980 mL of n-heptane and 2.2 L of ethanol were injected, and the reaction solution was stirred at 60°C for 1 hour, and cooled to room temperature, followed by filtration to obtain 263.3 g of sodium 2-(tetradecyloxy)acetate compound.

(¹H NMR (CD₃OD, 400 MHz) δ 3.83 (s, 2H), 3.48 (t, *J* = 6.9 Hz, 2H), 1.65 ~ 1.57 (m, 2H), 1.37 ~ 1.29 (m, 22H), 0.90 (t, *J* = 6.9 Hz, 3H)).

### 2) Preparation of 2-(tetradecyloxy)acetic acid (EPS-acid)

To the reaction vessel, 263.3 g of sodium 2-(tetradecyloxy)acetate prepared in 1) was added to 2.2 L of ethyl acetate and stirred, and then 1.45 L of 2M HCl aqueous solution was injected and stirred at room temperature for 2 hours. The resulting product was subjected to layer separation to obtain an organic layer, and then washed twice with 1.45 L of purified water. The obtained organic layer was washed with 1.45 L of saturated aqueous sodium chloride solution. After obtaining the organic layer, it was dried over anhydrous sodium sulfate, filtered, and concentrated under reduced pressure. The concentrated compound was dissolved in 1.63 L of n-hexane, and then the mixture was stirred at 0°C for 1 hour. The precipitated solid was filtered to obtain 110.6 g of 2-(tetradecyloxy)acetic acid compound.

(¹H NMR (CDCl₃, 400 MHz) δ 4.11 (s, 2H), 3.56 (t, *J* = 6.7 Hz, 2H), 1.66 ~ 1.59 (m, 2H), 1.36 ~ 1.25 (m, 22H), 0.88 (t, *J* = 7.2 Hz, 3H)).

### 3) Preparation of 2-(tetradecyloxy)acetyl chloride (EPS-Cl)

To the reaction vessel, 50.0 g of 2-(tetradecyloxy)acetic acid prepared in the above '2)' was added and dissolved by injecting 500 mL of dichloromethane and 0.5 mL of dimethylformamide. Into the reaction solution, 16.5 mL of oxalyl chloride was injected and stirred at room temperature for 3 hours. After the reaction was completed, the solvent was removed by distillation under reduced pressure to obtain 53.4 g of 2-(tetradecyloxy)acetyl chloride compound.

(¹H NMR (CDCl₃, 400 MHz) δ 4.39 (s, 2H), 3.56 (t, *J* = 6.6 Hz, 2H), 1.64 ~ 1.57 (m, 2H), 1.36 ~ 1.25 (m, 22H), 0.88 (t, *J* = 6.8 Hz, 3H)).

### [Example 2]

### Preparation of donepezil ether palmitate (DEP) [2-((1-benzylpiperidin-4-yl)methyl)-5,6-dimethoxy-1H-inden-3-yl 2-(tetradecyloxy)acetate]

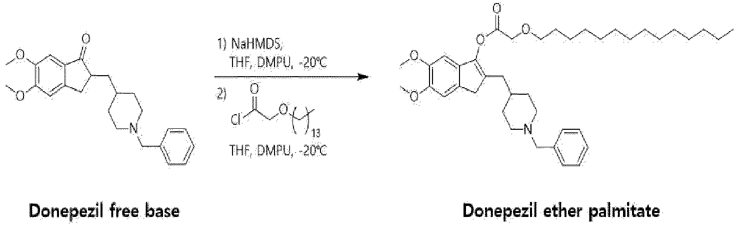

To the reaction vessel, 63.3 g of donepezil free base (purchased from Jinan Chenghui-Shuangda Chemical Co., Ltd) was added, and then 190 mL of tetrahydrofuran and 127 mL of DMPU (1,3-dimethyl-3,4,5,6-tetrahydro-2(1*H*)-pyrimidinone) were injected, dissolved, and cooled to -20°C. Into the reaction solution, 183.5 mL of NaHMDS (1.0M tetrahydrofuran solution) was injected for 30 minutes, and the mixture was stirred at -20°C for 1 hour. The reaction solution was injected into a solution in which 53.4 g of 2-(tetradecyloxy)acetyl chloride prepared in 'Example 1-3)' was dissolved in 380 mL of tetrahydrofuran and 253 mL of DMPU (1,3-dimethyl-3,4,5,6-tetrahydro-2(1*H*)-pyrimidinone) at -20°C for 40 minutes and stirred for 1 hour at the same temperature.

Into the reaction solution, 630 mL of saturated aqueous ammonium chloride solution was injected, filtered through celite, then washed with 630 mL of ethyl acetate, and filtered. The resulting product was subjected to layer separation to obtain an organic layer, and the aqueous layer was back-extracted with 630 mL of ethyl acetate. The combined organic layer was washed three times with 630 mL of 5% aqueous sodium chloride solution. The organic layer was dried over anhydrous sodium sulfate, filtered, and concentrated under reduced pressure. Into the concentrated solution, 630 mL of n-hexane was injected and stirred at 40°C, and the temperature was lowered back to room temperature and the slurry was stirred for 1 hour. The solution was filtered and the resulting filtrate was concentrated under reduced pressure. The concentrated solution was purified with a silica column and recrystallized with 630 mL of n-heptane to obtain 33.1 g of donepezil ether palmitate.

(¹H NMR (CDCl₃, 400 MHz) δ 7.32 ~ 7.21 (m, 5H), 6.96 (s, 1H), 6.59 (s, 1H), 4.37 (s, 2H), 3.88 (s, 3H), 3.87 (s, 3H), 3.64 (t, *J* = 6.7 Hz, 2H), 3.47 (s, 2H), 3.25 (s, 2H), 2.87 ~ 2.84 (m, 2H), 2.27 (d, *J* = 7.1 Hz, 2H), 1.90 (t, *J* = 10.7 Hz, 2H), 1.71 ~ 1.63 (m, 4H), 1.55 ~ 1.44 (m, 1H), 1.41 ~ 1.25 (m, 24H), 0.88 (t, ***J*** = 6.8 Hz, 3H)).

### [Example 3]

### Synthesis of 2-(dodecyloxy)acetyl chloride (EMS-Cl)

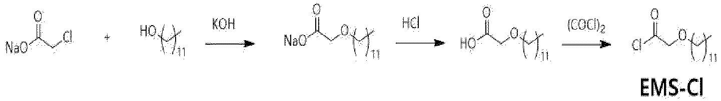

### 1) Preparation of sodium 2-(dodecyloxy)acetate (EMS-Na)

To the reaction vessel, 96.0 g of 1-dodecanol was added and dissolved at 60°C, and then 30.0 g of sodium chloroacetate and 30 mL of n-heptane were added. To the reaction solution, 22.8 g of potassium hydroxide was added, and the mixture was heated to 85°C and stirred for 2 hours. Into the reaction solution, 270 mL of n-heptane and 600 mL of ethanol were injected, stirred at 60°C for 20 minutes, and cooled to room temperature, followed by filtration to obtain 69.2 g of sodium 2-(dodecyloxy)acetate compound.

(¹H NMR (CD₃OD, 400 MHz) δ 3.83 (s, 2H), 3.47 (t, *J* = 6.9 Hz, 2H), 1.64 ~ 1.57 (m, 2H), 1.37 ~ 1.29 (m, 18H), 0.90 (t, *J* = 6.9 Hz, 3H)).

### 2) Preparation of 2-(dodecyloxy)acetic acid (EMS-acid)

To the reaction vessel, 68.0 g of sodium 2-(dodecyloxy)acetate prepared in the above '1)' was added to 904 mL of ethyl acetate and stirred, and then 904 mL of 2M HCl aqueous solution was injected and stirred at room temperature for 1 hour. The resulting product was subjected to layer separation to obtain an organic layer, and washed twice with 904 mL of purified water. The obtained organic layer was washed with 904 mL of saturated aqueous sodium chloride solution. After obtaining organic layer, it was dried over anhydrous sodium sulfate, filtered, and concentrated under reduced pressure. The concentrated compound was dissolved in 300 mL of n-hexane, and then the mixture was stirred at 0°C for 1 hour. The precipitated solid was filtered and washed with 60 mL of cooled n-hexane to obtain 36.2 g of 2-(dodecyloxy)acetic acid compound.

(¹H NMR (CDCl₃, 400 MHz) δ4.11 (s, 2H), 3.56 (t, *J* = 6.7 Hz, 2H), 1.66 ~ 1.59 (m, 2H), 1.36 ~ 1.25 (m, 18H), 0.87 (t, *J* = 6.9 Hz, 3H)).

### 3) Preparation of 2-(dodecyloxy)acetyl chloride (EMS-Cl)

To the reaction vessel, 35.4 g of 2-(dodecyloxy)acetic acid prepared in the above '2)' was added and dissolved by injecting 354 mL of dichloromethane and 0.35 mL of dimethylformamide. Into the reaction solution, 14.9 mL of oxalyl chloride was injected and stirred at room temperature for 2 hours. After the reaction was completed, the solvent was removed by distillation under reduced pressure to obtain 38.1 g of 2-(dodecyloxy)acetyl chloride compound.

(¹H NMR (CDCl₃, 400 MHz) δ 4.39 (s, 2H), 3.57 (t, *J* = 6.6 Hz, 2H), 1.64 ~ 1.57 (m, 2H), 1.36 ~ 1.26 (m, 18H), 0.88 (t, *J* = 6.8 Hz, 3H)).

### [Example 4]

### Preparation of donepezil ether myristate (DEM) [2-((1-benzylpiperidin-4-yl)methyl)-5,6-dimethoxy-1H-inden-3-yl 2-(dodecyloxy)acetate]

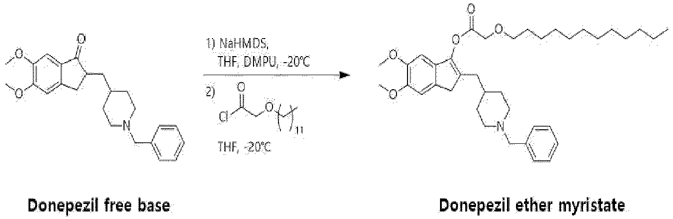

To the reaction vessel, 50.0 g of donepezil free base (purchased from Jinan Chenghui-Shuangda Chemical Co., Ltd) was added, and then 150 mL of tetrahydrofuran and 100 mL of DMPU (1,3-dimethyl-3,4,5,6-tetrahydro-2(1*H*)-pyrimidinone) were injected, dissolved, and cooled to -20°C. Into the reaction solution, 145 mL of NaHMDS (1.0M tetrahydrofuran solution) was injected for 30 minutes, and the mixture was stirred at -20°C for 1 hour. The reaction solution was injected for 30 minutes into a solution in which 38.1 g of 2-(dodecyloxy)acetyl chloride prepared in the above 'Example 3-3)' was dissolved in 500 mL of tetrahydrofuran at -20°C, and stirred for 30 minutes at the same temperature.

Into the reaction solution, 500 mL of saturated aqueous ammonium chloride solution was injected, 100 g of Celite was added, and the mixture was stirred for 10 minutes. The reaction solution was filtered and washed with 500 mL of ethyl acetate and filtered. The resulting product was subjected to layer separation to obtain an organic layer, and the aqueous layer was back-extracted with 500 mL of ethyl acetate. The combined organic layer was washed three times with 500 mL of 5% aqueous sodium chloride solution. The obtained organic layer was dried over anhydrous sodium sulfate, filtered, and concentrated under reduced pressure. Into the concentrated solution, 500 mL of n-hexane was injected, and the slurry was stirred. The solution was filtered and the resulting filtrate was concentrated under reduced pressure. The concentrated solution was purified with a silica column and recrystallized with 750 mL of n-heptane to obtain 26.7 g of donepezil ether myristate.

(¹H NMR (CDCl₃, 400 MHz) δ 7.32 ~ 7.21 (m, 5H), 6.96 (s, 1H), 6.59 (s, 1H), 4.37 (s, 2H), 3.87 (s, 3H), 3.87 (s, 3H), 3.64 (t, *J* = 6.7 Hz, 2H), 3.47 (s, 2H), 3.25 (s, 2H), 2.87 ~ 2.84 (m, 2H), 2.27 (d, *J* = 7.1 Hz, 2H), 1.91 (t, *J* = 10.8 Hz, 2H), 1.71 ~ 1.63 (m, 4H), 1.55 ~ 1.44 (m, 1H), 1.41 ~ 1.25 (m, 20H), 0.88 (t, *J* = 6.8 Hz, 3H)).

### [Example 5]

### Synthesis of 2-(tridecyloxy)acetyl chloride (EPDS-Cl)

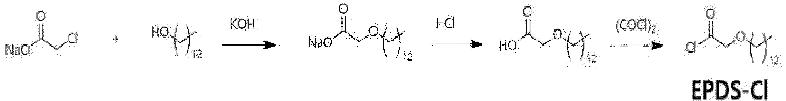

### 1) Preparation of sodium 2-(tridecyloxy)acetate (EPDS-Na)

To the reaction vessel, 100.0 g of 1-tridecanol was added and dissolved at 60°C, and then 29.1 g of sodium chloroacetate and 30 mL of n-heptane were added. To the reaction solution, 21.0 g of potassium hydroxide was added, and the mixture was heated to 85°C and stirred for 3 hours. Into the reaction solution, 262 mL of n-heptane and 581 mL of ethanol were injected, cooled to room temperature, and stirred for 16 hours. The solution was filtered to obtain 73.1 g of sodium 2-(tridecyloxy)acetate compound.

(¹H NMR (CD₃OD, 400 MHz) δ 3.83 (s, 2H), 3.47 (t, *J* = 6.9 Hz, 2H), 1.64 ~ 1.57 (m, 2H), 1.37 ~ 1.29 (m, 20H), 0.90 (t, *J* = 6.8 Hz, 3H)).

### 2) Preparation of 2-(tridecyloxy)acetic acid (EPDS-acid)

To the reaction vessel, 72.2 g of sodium 2-(tridecyloxy)acetate prepared in the above '1)' was added to 386 mL of ethyl acetate and stirred, and then 386 mL of 2M HCl aqueous solution was injected and stirred at room temperature for 1 hour. To the reaction solution, 145 mL of ethyl acetate was additionally added, and the mixture was stirred at room temperature for 2 hours. The resulting product was subjected to layer separation to obtain an organic layer, and washed twice with 386 mL of purified water. The obtained organic layer was washed with 386 mL of 10% aqueous sodium chloride solution. After obtaining organic layer, it was dried over anhydrous sodium sulfate, filtered, and concentrated under reduced pressure. The concentrated product was dissolved in 290 mL of n-hexane at 40°C, and the mixture was stirred at room temperature for 1 hour and stirred again at 0 to 5°C for 1 hour. The precipitated solid was filtered and washed with 60 mL of cooled n-hexane to obtain 41.3 g of 2-(tridecyloxy)acetic acid compound.

(¹H NMR (CDCl₃, 400 MHz) δ 4.10 (s, 2H), 3.56 (t, *J* = 6.7 Hz, 2H), 1.66 ~ 1.59 (m, 2H), 1.37 ~ 1.26 (m, 20H), 0.88 (t, *J* = 6.8 Hz, 3H)).

### 3) Preparation of 2-(tridecyloxy)acetyl chloride (EPDS-Cl)

To the reaction vessel, 41.0 g of 2-(tridecyloxy) acetic acid prepared in the above '2)' was added and dissolved by injecting 410 mL of dichloromethane and 0.4 mL of dimethylformamide. Into the reaction solution, 16.3 mL of oxalyl chloride was injected and stirred at room temperature for 2 hours. After the reaction was completed, the solvent was removed by distillation under reduced pressure to obtain 43.9 g of 2-(tridecyloxy)acetyl chloride compound.

(¹H NMR (CDCl₃, 400 MHz) δ 4.39 (s, 2H), 3.57 (t, *J* = 6.6 Hz, 2H), 1.64 ~ 1.57 (m, 2H), 1.36 ~ 1.26 (m, 20H), 0.88 (t, *J* = 6.8 Hz, 3H)).

### [Example 6]

### Preparation of donepezil ether pentadecanoate (DEPD) [2-((1-benzylpiperidin-4-yl)methyl)-5,6-dimethoxy-1H-inden-3-yl 2-(tridecyloxy)acetate]

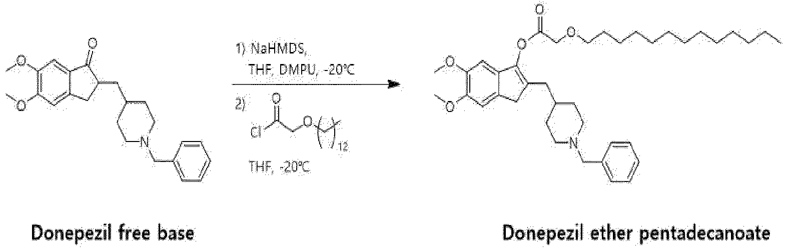

To the reaction vessel, 54.8 g of donepezil free base (purchased from Jinan Chenghui-Shuangda Chemical Co., Ltd) was added, and then 164 mL of tetrahydrofuran and 110 mL of DMPU (1,3-dimethyl-3,4,5,6-tetrahydro-2(1*H*)-pyrimidinone) were injected, dissolved, and cooled to -20°C. Into the reaction solution, 159 mL of NaHMDS (1.0M tetrahydrofuran solution) was injected for 30 minutes, and the mixture was stirred at -20°C for 1 hour. The reaction solution was injected for 30 minutes into a solution in which 43.9 g of 2-(tridecyloxy)acetyl chloride prepared in 'Example 5-3)' was dissolved in 548 mL of tetrahydrofuran at -20°C, and stirred for 30 minutes at the same temperature.

Into the reaction solution, 548 mL of saturated aqueous ammonium chloride solution was injected, 110 g of Celite was added, and the mixture was stirred for 10 minutes. The reaction solution was filtered and washed with 548 mL of ethyl acetate and filtered. The resulting product was subjected to layer separation to obtain an organic layer, and the aqueous layer was back-extracted with 548 mL of ethyl acetate. The combined organic layer was washed three times with 548 mL of 5% aqueous sodium chloride solution. The obtained organic layer was dried over anhydrous sodium sulfate, filtered, and concentrated under reduced pressure. Into the concentrated solution, 548 mL of n-hexane was injected, and the slurry was stirred. The solution was filtered and the resulting filtrate was concentrated under reduced pressure. The concentrated solution was purified with a silica column and recrystallized with 1.37 L of n-heptane to obtain 27.2 g of donepezil ether pentadecanoate.

(¹H NMR (CDCl₃, 400 MHz) δ 7.32 ~ 7.21 (m, 5H), 6.96 (s, 1H), 6.59 (s, 1H), 4.37 (s, 2H), 3.87 (s, 3H), 3.87 (s, 3H), 3.64 (t, *J* = 6.7 Hz, 2H), 3.47 (s, 2H), 3.25 (s, 2H), 2.87 ~ 2.84 (m, 2H), 2.27 (d, *J* = 7.1 Hz, 2H), 1.91 (t, *J* = 10.8 Hz, 2H), 1.71 ~ 1.63 (m, 4H), 1.55 ~ 1.44 (m, 1H), 1.43 ~ 1.25 (m, 22H), 0.88 (t, *J* = 6.8 Hz, 3H)).

### [Example 7]

### Synthesis of 2-(pentadecyloxy)acetyl chloride (EHDS-Cl)

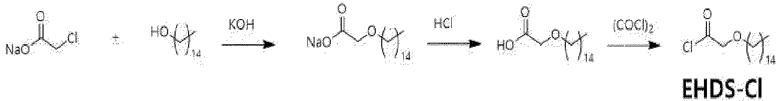

### 1) Preparation of sodium 2-(pentadecyloxy)acetate (EHDS-Na)

To the reaction vessel, 200 g of 1-pentadecanol was added and dissolved at 70°C to 80°C, and 51 mL of n-heptane was injected. To the reaction solution, 51.0 g of sodium chloroacetate and 36.8 g of potassium hydroxide were added. The reaction solution was heated to 90°C and stirred for 4 hours. Into the reaction solution, 1 L of ethanol and 450 mL of n-heptane were injected, and the mixture was stirred at 60°C for 1 hour. The reaction solution was cooled to room temperature again and stirred for 15 hours. The reaction solution was filtered and washed with a mixture of 150 mL of ethanol and 75 mL of n-heptane to obtain 130.7 g of sodium 2-(pentadecyloxy)acetate compound.

(¹H NMR (CD₃OD, 400 MHz) δ 3.83 (s, 2H), 3.47 (t, *J* = 6.9 Hz, 2H), 1.64 ~ 1.57 (m, 2H), 1.37 ~ 1.29 (m, 24H), 0.90 (t, *J* = 6.9 Hz, 3H)).

### 2) Preparation of 2-(pentadecyloxy)acetic acid (EHDS-acid)

To the reaction vessel, 130.4 g of sodium 2-(pentadecyloxy)acetate prepared in the above '1)' was added to 678 mL of ethyl acetate and stirred, and then 678 mL of 2M HCl aqueous solution was injected and stirred at room temperature for 1 hour. To the reaction solution, 255 mL of ethyl acetate was additionally added, and the mixture was stirred at room temperature for 1 hour. The resulting product was subjected to layer separation to obtain an organic layer, and washed twice with 678 mL of purified water. The obtained organic layer was washed with 678 mL of saturated aqueous sodium chloride solution. Then, the obtained organic layer was dried over anhydrous sodium sulfate, filtered, and concentrated under reduced pressure. The concentrated compound was dissolved in 800 mL of n-hexane at 40°C and then the mixture was stirred at room temperature for 1 hour. The reaction solution was stirred again at 0°C for 1 hour. The precipitated solid was filtered and washed with 150 mL of cooled n-hexane to obtain 72.6 of 2-(pentadecyloxy)acetic acid compound.

(¹H NMR (CDCl₃, 400 MHz) δ4.11 (s, 2H), 3.56 (t, *J* = 6.7 Hz, 2H), 1.69 ~ 1.56 (m, 2H), 1.37 ~ 1.25 (m, 24H), 0.88 (t, *J* = 6.8 Hz, 3H)).

### 3) Preparation of 2-(pentadecyloxy)acetyl chloride (EHDS-Cl)

To the reaction vessel, 72.0 g of 2-(pentadecyloxy)acetic acid prepared in the above '2)' was added and dissolved by injecting 720 mL of dichloromethane and 0.7 mL of dimethylformamide. Into the reaction solution, 25.9 mL of oxalyl chloride was injected and stirred at room temperature for 2 hours. After the reaction was completed, the solvent was removed by distillation under reduced pressure to obtain 76.6 g of 2-(dodecyloxy)acetyl chloride compound.

(¹H NMR (CDCl₃, 400 MHz) δ 4.39 (s, 2H), 3.57 (t, *J* = 6.6 Hz, 2H), 1.64 ~ 1.57 (m, 2H), 1.39 ~ 1.25 (m, 24H), 0.88 (t, *J* = 6.8 Hz, 3H)).

### [Example 8]

### Preparation of donepezil ether heptadecanoate (DEHD) [2-((1-benzylpiperidin-4-yl)methyl)-5,6-dimethoxy-1H-inden-3-yl 2-(pentadecyloxy)acetate]

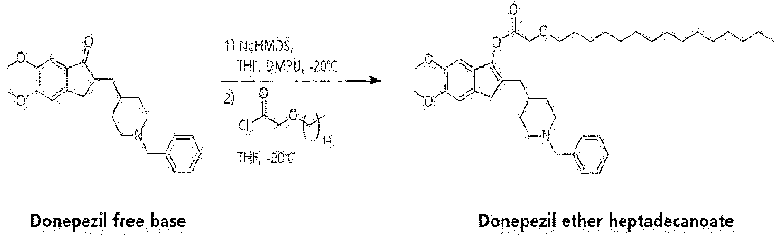

To the reaction vessel, 86.7 g of donepezil free base (purchased from Jinan Chenghui-Shuangda Chemical Co., Ltd) was added, and then 260 mL of tetrahydrofuran and 173 mL of DMPU (1,3-dimethyl-3,4,5,6-tetrahydro-2(1*H*)-pyrimidinone) were injected, dissolved, and cooled to -20°C. Into the reaction solution, 251 mL of NaHMDS (1.0M tetrahydrofuran solution) was injected for 30 minutes, and the mixture was stirred at -20°C for 1 hour and heated to room temperature. The reaction solution was injected for 30 minutes into a solution in which 76.6 g of 2-(pentadecyloxy)acetyl chloride prepared in the above 'Example 7-3)' was dissolved in 870 mL of tetrahydrofuran at -20°C, and stirred for 30 minutes at the same temperature.

Into the reaction solution, 867 mL of saturated aqueous ammonium chloride solution was injected, filtered through celite, then washed with 867 mL of ethyl acetate, and filtered. The resulting product was subjected to layer separation to obtain an organic layer, and the aqueous layer was back-extracted with 867 mL of ethyl acetate. The combined organic layer was washed three times with 867 mL of 5% aqueous sodium chloride solution. The obtained organic layer was dried over anhydrous sodium sulfate, filtered, and concentrated under reduced pressure. Into the concentrated solution, 867 mL of n-hexane was injected and stirred at 40°C, and the temperature was lowered back to room temperature and the slurry was stirred for 1 hour. The solution was filtered and the resulting filtrate was concentrated under reduced pressure. The concentrated solution was purified with a silica column and recrystallized with 2.6 L of n-heptane to obtain 55.2 g of donepezil ether heptadecanoate.

(¹H NMR (CDCl₃, 400 MHz) δ 7.32 ~ 7.21 (m, 5H), 6.96 (s, 1H), 6.59 (s, 1H), 4.37 (s, 2H), 3.87 (s, 3H), 3.87 (s, 3H), 3.64 (t, *J* = 6.7 Hz, 2H), 3.47 (s, 2H), 3.25 (s, 2H), 2.87 ~ 2.84 (m, 2H), 2.27 (d, *J* = 7.1 Hz, 2H), 1.91 (t, *J* = 10.8 Hz, 2H), 1.71 ~ 1.63 (m, 4H), 1.53 ~ 1.45 (m, 1H), 1.41 ~ 1.25 (m, 26H), 0.88 (t, *J* = 6.8 Hz, 3H)).

### [Example 9]

### Synthesis of 2-(hexadecyloxy)acetyl chloride (ESS-Cl)

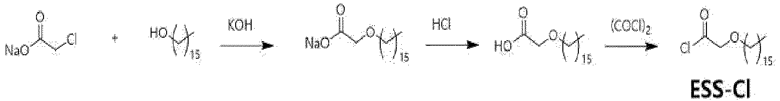

### 1) Preparation of sodium 2-(hexadecyloxy)acetate (ESS-Na)

To the reaction vessel, 104.1 g of 1-hexadecanol was added and dissolved at 60°C, and 25.0 g of sodium chloroacetate was added. The reaction solution was stirred for 10 minutes, and 25 mL of n-heptane and 19.0 g of potassium hydroxide were added. The reaction solution was heated to 80°C to 90°C and stirred for 3 hours. Into the reaction solution, 333 mL of ethanol and 167 mL of n-heptane were injected, and the mixture was stirred at 60°C for 20 minutes. The reaction solution was cooled to room temperature, filtered, and washed with a mixture of 167 mL of ethanol and 37 mL of n-heptane to obtain 70.9 g of sodium 2-(hexadecyloxy)acetate compound.

(¹H NMR (CD₃OD, 400 MHz) δ 3.83 (s, 2H), 3.47 (t, *J* = 6.8 Hz, 2H), 1.64 ~ 1.57 (m, 2H), 1.37 ~ 1.29 (m, 26H), 0.90 (t, *J* = 6.8 Hz, 3H)).

### 2) Preparation of 2-(hexadecyloxy)acetic acid (ESS-acid)

To the reaction vessel, 70.0 g of sodium 2-(hexadecyloxy)acetate prepared in the above '1)' was added to 1.4 L of ethyl acetate and stirred, and then 931 mL of 2M HCl aqueous solution was injected and stirred at room temperature for 1 hour. The resulting product was subjected to layer separation to obtain an organic layer, and washed twice with 931 mL of purified water. The obtained organic layer was washed with 931 mL of saturated aqueous sodium chloride solution. Then, the obtained organic layer was dried over anhydrous sodium sulfate, filtered, and concentrated under reduced pressure. The concentrated compound was dissolved in 900 mL of n-hexane at 40°C and then the mixture was stirred at room temperature for 1 hour. The reaction solution was stirred again at 0°C for 1 hour. The precipitated solid was filtered and washed with 210 mL of cooled n-hexane to obtain 44.7 g of 2-(hexadecyloxy)acetic acid compound.

(¹H NMR (CDCl₃, 400 MHz) δ4.10 (s, 2H), 3.57 (t, *J* = 6.7 Hz, 2H), 1.66 ~ 1.59 (m, 2H), 1.37 ~ 1.26 (m, 26H), 0.88 (t, *J* = 6.8 Hz, 3H)).

### 3) Preparation of 2-(hexadecyloxy)acetyl chloride (ESS-Cl)

To the reaction vessel, 44.3 g of 2-(hexadecyloxy)acetic acid prepared in the above '2)' was added and dissolved by injecting 880 mL of dichloromethane and 0.5 mL of dimethylformamide. Into the reaction solution, 15.2 mL of oxalyl chloride was injected and stirred at room temperature for 2 hours. After the reaction was completed, the solvent was removed by distillation under reduced pressure to obtain 47.0 g of 2-(hexadecyloxy)acetyl chloride compound.

(¹H NMR (CDCl₃, 400 MHz) δ 4.39 (s, 2H), 3.56 (t, *J* = 6.6 Hz, 2H), 1.64 ~ 1.57z (m, 2H), 1.36 ~ 1.25 (m, 26H), 0.88 (t, *J* = 6.8 Hz, 3H)).

### [Example 10]

### Preparation of donepezil ether stearate (DES) [2-((1-benzylpiperidin-4-yl)methyl)-5,6-dimethoxy-1H-inden-3-yl 2-(hexadecyloxy)acetate]

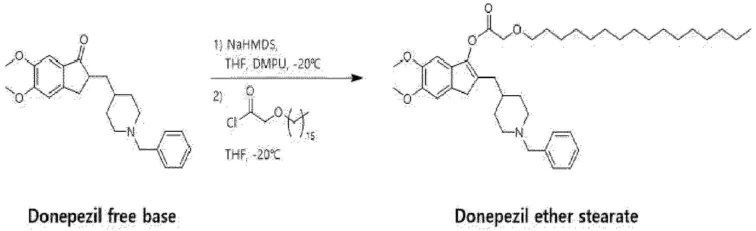

To the reaction vessel, 50.9 g of donepezil free base (purchased from Jinan Chenghui-Shuangda Chemical Co., Ltd) was added, and then 152 mL of tetrahydrofuran and 102 mL of DMPU (1,3-dimethyl-3,4,5,6-tetrahydro-2(1*H*)-pyrimidinone) were injected, dissolved, and cooled to -20°C. Into the reaction solution, 147 mL of NaHMDS (1.0M tetrahydrofuran solution) was injected for 30 minutes, and the mixture was stirred at -20°C for 1 hour and heated to room temperature. The reaction solution was injected for 30 minutes into a solution in which 47.0 g of 2-(hexadecyloxy)acetyl chloride prepared in the above 'Example 9-3)' was dissolved in 508 mL of tetrahydrofuran at -20°C, and stirred for 30 minutes at the same temperature.

Into the reaction solution, 509 mL of saturated aqueous ammonium chloride solution was injected, filtered through celite, then washed with 500 mL of ethyl acetate, and filtered. The resulting product was subjected to layer separation to obtain an organic layer, and the aqueous layer was back-extracted with 500 mL of ethyl acetate. The combined organic layer was washed three times with 509 mL of 5% aqueous sodium chloride solution. The obtained organic layer was dried over anhydrous sodium sulfate, filtered, and concentrated under reduced pressure. Into the concentrated solution, 509 mL of n-hexane was injected and stirred at 40°C, and the temperature was lowered back to room temperature and the slurry was stirred for 1 hour. The solution was filtered and the resulting filtrate was concentrated under reduced pressure. The concentrated solution was purified with a silica column and recrystallized with 1.5 L of n-heptane to obtain 28.7 g of donepezil ether stearate.

(¹H NMR (CDCl₃, 400 MHz) δ 7.32 ~ 7.21 (m, 5H), 6.96 (s, 1H), 6.59 (s, 1H), 4.38 (s, 2H), 3.87 (s, 3H), 3.87 (s, 3H), 3.64 (t, *J* = 6.7 Hz, 2H), 3.47 (s, 2H), 3.25 (s, 2H), 2.87 ~ 2.84 (m, 2H), 2.27 (d, *J* = 7.1 Hz, 2H), 1.91 (t, *J* = 10.9 Hz, 2H), 1.71 ~ 1.63 (m, 4H), 1.52 ~ 1.45 (m, 1H), 1.41 ~ 1.25 (m, 28H), 0.88 (t, *J* = 6.7 Hz, 3H)).

### [Example 11]

### Preparation of donepezil compositions

Solution phase compositions were prepared using donepezil (D), Example 2 (DEP), Example 4 (DEM), Example 6 (DEPD), Example 8 (DEHD) and Example 10 (DES), each having a structure in Table 1 below.

**[Table 1]**

| Type | Structure |
|---|---|
| Donepezil (D), 2-((1-benzylpiperidin-4-yl)methyl)-5,6-dimethoxy-2,3-dihydro-1H-inden-1-one | 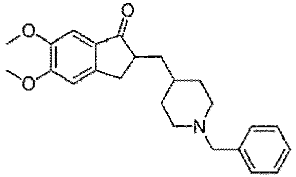 |
| Example 2 (DEP) | 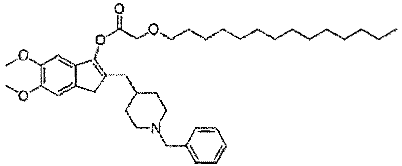 |
| Example 4 (DEM) | 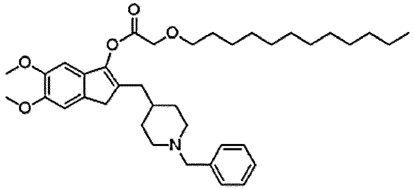 |
| Example 6 (DEPD) | 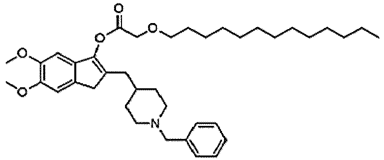 |
| Example 8 (DEHD) | 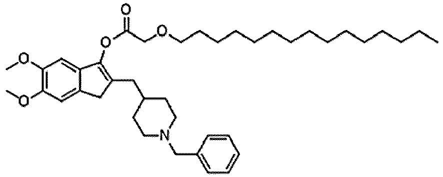 |
| Example 10 (DES) | 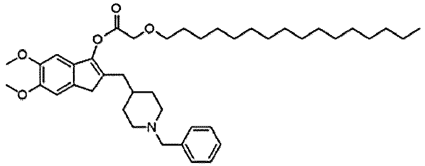 |

The main ingredients of Table 1 above, castor oil, and benzyl benzoate were mixed according to the components and amounts of Table 2 below, and stirred at room temperature for 0.5 to 3 hours to prepare solution phase compositions.

**[Table 2]**

| (Unit: mg) | D | DEP | DEM | DEPD | DEHD | DES |
|---|---|---|---|---|---|---|
| Main ingredient * | 137 | 229 | 219 | 224 | 234 | 239 |
| Castor oil | 1,200 | 1,200 | 1,200 | 1,200 | 1,200 | 1,200 |
| Benzyl benzoate | 1, 800 | 1, 800 | 1, 800 | 1, 800 | 1, 800 | 1, 800 |

| | | | | | | |
|---|---|---|---|---|---|---|
| * indicates donepezil and corresponds to an equivalent dose | | | | | | |

### [Experimental Example 1]

### Pharmacokinetic evaluation

Four male SD rats with an average weight of 300 g were intramuscularly injected with a dose of 4 mg/kg of donepezil in the formulation of donepezil (D) and 40 mg/kg of donepezil each in the DEP, DEM, DEPD, DEHD, and DES formulations, and the concentrations of donepezil in plasma samples of SD rats were analyzed using LC-MS/MS.

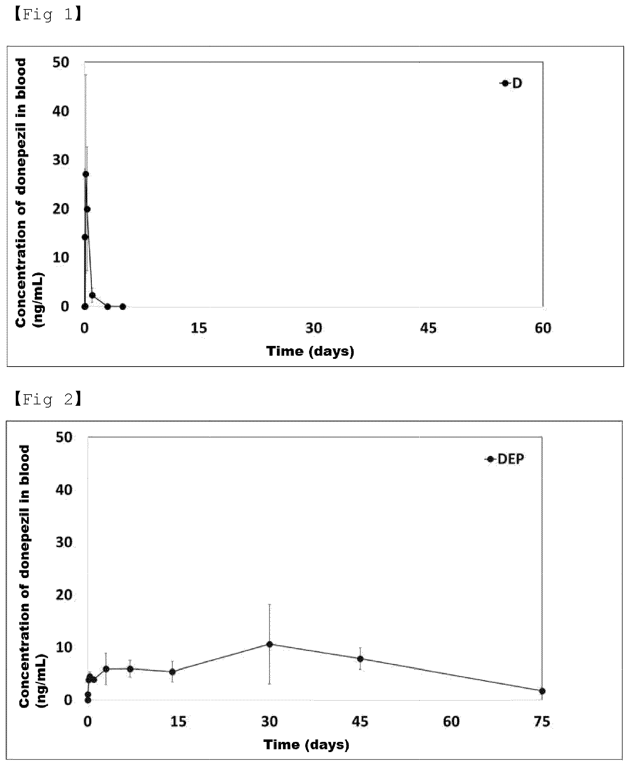

As a result of the analysis, the release pattern when DEP was administered among the DEP, DEM, DEPD, DEHD and DES formulations was most preferred for the sustained-release composition, and the results for donepezil and DEP are shown in Table 3 and FIGS. 1 and 2 below.

**[Table 3]**

| | D | DEP |
|---|---|---|
| Cmax (ng/mL) | 27.5 | 7.9 |
| AUCₗₐₛₜ (ngh/mL) | 367.6 | 6290.0 |

As shown in Table 3 and FIG. 1, it was confirmed that when the donepezil (D) composition was administered, the drug was rapidly released immediately after administration, and the Cmax was as high as 27.5 ngh/mL, indicating a high possibility of causing side effects or toxicity. Further, the release of the drug was terminated within 3 days, confirming that the donepezil composition was not suitable as a sustained-release composition.

On the other hand, as shown in Table 3 and FIG. 2, it was confirmed that when the composition of Example 2 (DEP) was administered, even though donepezil was administered in an amount about 10 times, Cmax was as low as 7.9 ngh/mL without rapid release of drug after administration, and a difference between Cmax and maintenance concentration was small. Further, unlike the case where the donepezil (D) composition was administered, it was confirmed that the DEP composition maintained an effective blood concentration for a period of 8 weeks or longer even with a single administration.

As a result, it may be appreciated that donepezil ether palmitate (DEP) exhibits a better release pattern than that of donepezil. It may be confirmed that when DEP is administered into the body, maintaining sustained-release for a long period of time is possible without rapid release of the drug after administration, thereby having excellent properties such as minimizing the risk of side effects including toxic reactions while maintaining an effective therapeutic drug concentration for a long time even with a single administration.

## Claims

1. A compound represented by the following Chemical Formula 1 or a pharmaceutically acceptable salt thereof: 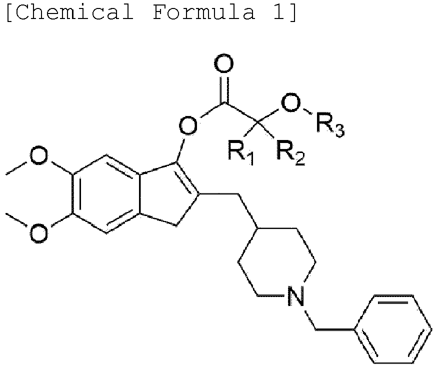
in the Formula above,
R₁ and R₂ are each independently hydrogen, halogen, hydroxy, C₁-C₆ alkyl, C₁-C₆ haloalkyl, C₁-C₆ alkoxy, or aryl; and
R₃ is C₁₂-C₁₆ alkyl.

2. 2-((1-Benzylpiperidin-4-yl)methyl)-5,6-dimethoxy-1H-inden-3-yl 2-(tetradecyloxy)acetate represented by the following Chemical Formula 2 or a pharmaceutically acceptable salt thereof:

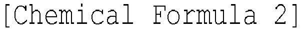

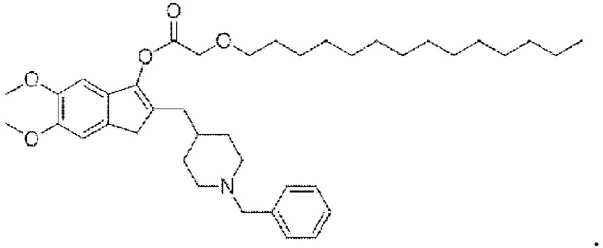

3. A sustained-release pharmaceutical composition for preventing or treating dementia, comprising a compound represented by the following Chemical Formula 1 or a pharmaceutically acceptable salt thereof: 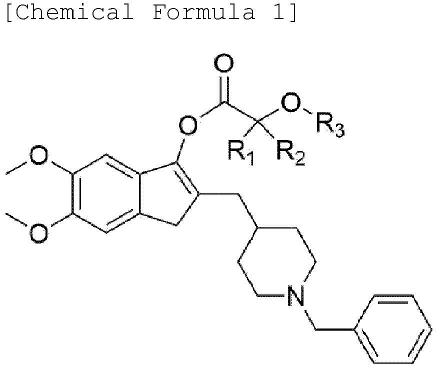
in the Formula above,
R₁ and R₂ are each independently hydrogen, halogen, hydroxy, C₁-C₆ alkyl, C₁-C₆ haloalkyl, C₁-C₆ alkoxy, or aryl; and
R₃ is C₁₂-C₁₆ alkyl.

4. A sustained-release pharmaceutical composition for preventing or treating dementia, comprising a compound represented by the following Chemical Formula 2 or a pharmaceutically acceptable salt thereof:

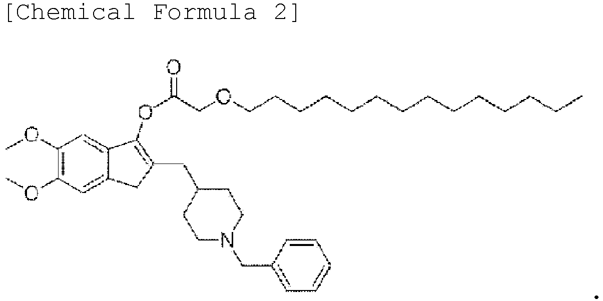

5. The sustained-release pharmaceutical composition of claim 3 or 4, wherein the composition is for injection administered every 2 to 20 weeks.

6. The sustained-release pharmaceutical composition of claim 3 or 4, wherein the composition is for intramuscular injection.

7. A method for preparing 2-((1-benzylpiperidin-4-yl)methyl)-5,6-dimethoxy-1H-inden-3-yl 2-(tetradecyloxy)acetate, comprising reacting donepezil free base with 2-(tetradecyloxy)acetyl chloride.

8. The method for preparing 2-((1-benzylpiperidin-4-yl)methyl)-5,6-dimethoxy-1H-inden-3-yl 2-(tetradecyloxy)acetate of claim 7, wherein the 2-(tetradecyloxy)acetyl chloride is prepared by comprising:
(i) mixing 1-tetradecanol, sodium chloroacetate, and potassium hydroxide to obtain sodium 2-(tetradecyloxy)acetate;
(ii) reacting the sodium 2-(tetradecyloxy)acetate with an aqueous HCl solution to obtain 2-(tetradecyloxy)acetic acid; and
(iii) reacting the 2-(tetradecyloxy)acetic acid with oxalyl chloride.
